# EUROPEAN PATENT SPECIFICATION

(11) **EP 4 505 965 B1**
(45) Date of publication and mention of the grant of the patent: **22.04.2026**
(21) Application number: 24193809.1
(22) Date of filing: 09.08.2024
(51) Int. Cl.: A61B 34/30, A61B 34/00, A61B 90/00

(54) **SURGICAL ROBOTIC SYSTEM FOR DETECTION AND PREDICTION OF CABLE BREAKAGE**
CHIRURGISCHES ROBOTERSYSTEM ZUR ERKENNUNG UND VORHERSAGE EINES KABELBRUCHS
SYSTÈME ROBOTIQUE CHIRURGICAL DE DÉTECTION ET DE PRÉDICTION DE RUPTURE DE CÂBLE

(30) Priority: 11.08.2023 US 202363518902 P; 09.11.2023 US 202363597489 P; 03.07.2024 US 202418762802
(43) Date of publication of application: 12.02.2025
(73) Proprietor: Covidien LP, Mansfield, MA 02048 (US)
(72) Inventor: MORADI DALVAND, Mohsen, Boston, 02210 (US); BALTER, Max L., Boston, 02210 (US)
(74) Representative: Froud, Christopher Andrew

(56) References cited:
- WO-A1-2021/183152
- WO-A1-2021/252199
- WO-A1-2022/060565
- US-A1- 2019 274 769

## Description

### CROSS-REFERENCE TO RELATED APPLICATIONS

This application claims the benefit of, and priority to, U.S. Provisional Patent Application Serial No. 63/518,902 filed on August 11, 2023, and U.S. Provisional Patent Application Serial No. 63/597,489 filed on November 9, 2023.

### BACKGROUND

Surgical robotic systems are currently being used in a variety of surgical procedures, including minimally invasive medical procedures. Some surgical robotic systems include a surgeon console controlling a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical port or a natural orifice of a patient to position the end effector at a work site within the patient's body.

Surgical robotic instruments are actuated by one or more cables, which may snap during use due to high strain placed on them during actuation. Cable snapping results in uncontrolled movement of portions of the instruments, e.g., end effector, which may damage other instruments or camera.

US2019274769A1 discloses a surgical robotic system according to the prior art for detecting cable failure.

### SUMMARY

The invention is defined in appended independent claim 1, further embodiments are described in the dependent claims.

According to one embodiment of the present disclosure, a surgical robotic system is disclosed. The system includes an instrument drive unit (IDU) having: a first high-side motor with a first high-side torque sensor for measuring a torque of the first high-side motor and a first high-side position sensor for measuring a position of the first high-side motor. The IDU also includes a first low-side motor having a first low-side torque sensor for measuring a torque of the first low-side motor and a first low-side position sensor for measuring a position of the first low-side motor. The IDU further includes a second high-side motor having a second high-side torque sensor for measuring a torque of the second high-side motor and a second high-side position sensor for measuring a position of the second high-side motor. The IDU additionally includes a second low-side motor having a second low-side torque sensor for measuring a torque of the second low-side motor and a second low-side position sensor for measuring a position of the second low-side motor. The system also includes an instrument having an end effector with a first jaw and a second jaw each of which is movable between an open position and a closed position. The instrument also includes a first high-side cable coupled to the first jaw and actuatable by the first high-side motor to move the first jaw toward the closed position; a first low-side cable coupled to the first jaw and actuatable by the first low-side motor to move the first jaw toward the open position; a second high-side cable coupled to the second jaw and actuatable by the second high-side motor to move the second jaw toward the closed position; and a second low-side cable coupled to the second jaw and actuatable by the second low-side motor to move the second jaw toward the closed position. The system also includes a controller for calculating an angle between the first jaw and the second jaw based on the position of one or more of the motors. The controller also calculates a combined torque of the first high-side motor and the second high-side motor. The controller also determines whether one of the first low-side cable or the second low-side cable is broken based on the angle between the first jaw and the second jaw and the combined torque. The controller additionally outputs an alert based on the determination that one of the first low-side cable or the second low-side cable is broken.

Implementations of the above embodiment may include one or more of the following features. According to one aspect of the above embodiment, the controller may further calculate a derivative of the angle between the first jaw and the second jaw. The controller may also calculate a first moving average of the derivative of the angle between the first jaw and the second jaw and a plurality of subsequent moving averages of the first moving average. The controller may additionally calculate a scaled value for the first moving average and each moving average of the plurality of subsequent moving averages. Each moving average of the plurality of subsequent moving averages may have a unique window size. The controller may also identify a minimum moving average of the derivative from the first moving average and each moving average of the plurality of subsequent moving averages. The controller may further compare the minimum moving average of the derivative to a threshold derivative of the angle between the first jaw and the second jaw. The controller may also determine whether one of the first low-side cable or the second low-side cable is broken based on the angle between the first jaw and the second jaw exceeding an angle threshold and the minimum moving average of the derivative exceeding the threshold derivative of the angle between the first jaw and the second jaw.

According to another embodiment of the present disclosure, a surgical robotic system is disclosed. The system includes an instrument drive unit (IDU) having a plurality of motors including a first pair motors and a second pair of motors, where each motor of the plurality of motors includes a torque sensor for measuring motor torque and a position sensor for measuring angular position. The system also includes an instrument coupled to the IDU, the instrument includes an end effector having a first jaw and a second jaw movable between an open position and a closed position. The instrument also includes a first pair of cables each of which is actuated by one motor of the first pair motors to move the first and second jaws toward the closed position, where one of the first pair of cables is coupled to the first jaw and another of the first pair of cables is coupled to the second jaw. The instrument also includes a second pair of cables each of which is actuated by one motor of the second pair motors to move the first and second jaws toward the open position, where one of the second pair of cables is coupled to the first jaw and another of the second pair of cables is coupled to the second jaw. The system further includes a controller for calculating an angle between the first jaw and the second jaw based on the angular position of one or more motors of the plurality of motors. The controller also calculates a combined torque of the first pair of motors and determines whether one or both cables of the second pair of cables is broken based on the angle between the first jaw and the second jaw and the combined torque. The controller also outputs an alert based on the determination that one or both cables of the second pair of cables is broken.

Implementations of the above embodiment may include one or more of the following features. According to one aspect of the above embodiment, the controller may further calculate a derivative of the angle between the first jaw and the second jaw. The controller may also calculate a first moving average of the derivative of the angle between the first jaw and the second jaw and a plurality of subsequent moving averages of the first moving average. The controller may additionally calculate a scaled value for the first moving average and each moving average of the plurality of subsequent moving averages. Each moving average of the plurality of subsequent moving averages has a unique window size. The controller may also identify a minimum moving average of the derivative from the first moving average and each moving average of the plurality of subsequent moving averages. The controller may additionally compare the minimum moving average of the derivative to a threshold derivative of the angle between the first jaw and the second jaw. The controller may further determine whether one or both cables of the second pair of cables is broken based on the combined torque of the first pair of motors exceeding a torque threshold and the minimum moving average of the derivative exceeding the threshold derivative of the angle between the first jaw and the second jaw.

According to a further embodiment of the present disclosure, a method for controlling a surgical robotic instrument is disclosed. The method includes moving apart a first jaw and a second jaw, where the first jaw is coupled to a first low-side cable actuatable by a first low-side motor and a second jaw is coupled to a second low-side cable actuatable by a second low-side motor. The method also includes approximating the first jaw and the second jaw, where the first jaw is coupled to a first high-side cable actuatable by a first high-side motor and the second jaw is coupled to a second high-side cable actuatable by a second high-side motor. The method further includes measuring torque of each of the first and second high-side motors and first and second low-side motors and measuring an angular position of each of the first and second high-side motors and first and second low-side motors. The method additionally includes calculating, at a controller, an angle between the first jaw and the second jaw based on the angular position of one of the first high-side motor, the second high-side motor, the first low-side motor, or the second low-side motor. The method also includes calculating, at the controller, a combined torque of the first high-side motor and the second high-side motor. The method further includes determining, at the controller, whether one of the first low-side cable or the second low-side cable is broken based on the angle between the first jaw and the second jaw and the combined torque. The method further includes outputting an alert based on the determination that one of the first low-side cable or the second low-side cable is broken.

Implementations of the above embodiment may include one or more of the following features. According to one aspect of the above embodiment, the method may include calculating, at the controller, a derivative of the angle between the first jaw and the second jaw and calculating, at the controller, a first moving average of the derivative of the angle between the first jaw and the second jaw and a plurality of subsequent moving averages of the first moving average. The method may also include identifying, at the controller, a minimum moving average of the derivative from the first moving average and each moving average of the plurality of subsequent moving averages and comparing, at the controller, the minimum moving average of the derivative to a threshold derivative of the angle between the first jaw and the second jaw. Determining whether one of the first low-side cable or the second low-side cable is broken based on the combined torque exceeding a torque threshold and the minimum moving average of the derivative exceeding the threshold derivative of the angle between the first jaw and the second jaw.

### BRIEF DESCRIPTION OF THE DRAWINGS

Various embodiments of the present disclosure are described herein with reference to the drawings wherein:
FIG. 1 is a schematic illustration of a surgical robotic system including a control tower, a console, and one or more surgical robotic arms each disposed on a movable cart according to an embodiment of the present disclosure;
FIG. 2 is a perspective view of a surgical robotic arm of the surgical robotic system of FIG. 1 according to an embodiment of the present disclosure;
FIG. 3 is a perspective view of a movable cart having a setup arm with the surgical robotic arm of the surgical robotic system of FIG. 1 according to an embodiment of the present disclosure;
FIG. 4 is a schematic diagram of a computer architecture of the surgical robotic system of FIG. 1 according to an embodiment of the present disclosure;
FIG. 5 is a plan schematic view of movable carts of FIG. 1 positioned about a surgical table according to an aspect of the present disclosure;
FIG. 6 is a perspective view, with parts separated, of an instrument drive unit and a surgical instrument according to an embodiment of the present disclosure;
FIG. 7 is a top, perspective view of an end effector, according to an embodiment of the present disclosure, for use in the surgical robotic system of FIG. 1;
FIG. 8 shows the end effector in various configurations according to an embodiment of the present disclosure;
FIG. 9 is a flow chart of a method for detection and prediction of high-side cable breakage according to an embodiment of the present disclosure; and
FIG. 10 is a flow chart of a method for detection and prediction of low-side cable breakage according to an embodiment of the present disclosure.

### DETAILED DESCRIPTION

Embodiments of the presently disclosed surgical robotic system are described in detail with reference to the drawings, in which like reference numerals designate identical or corresponding elements in each of the several views.

As will be described in detail below, the present disclosure is directed to a surgical robotic system, which includes a surgeon console, a control tower, and one or more movable carts having a surgical robotic arm coupled to a setup arm. The surgeon console receives user input through one or more interface devices. The input is processed by the control tower as movement commands for moving the surgical robotic arm and an instrument and/or camera coupled thereto. Thus, the surgeon console enables teleoperation of the surgical arms and attached instruments/camera. The surgical robotic arm includes a controller, which is configured to process the movement commands and to generate a torque commands for activating one or more actuators of the robotic arm, which would, in turn, move the robotic arm in response to the movement commands.

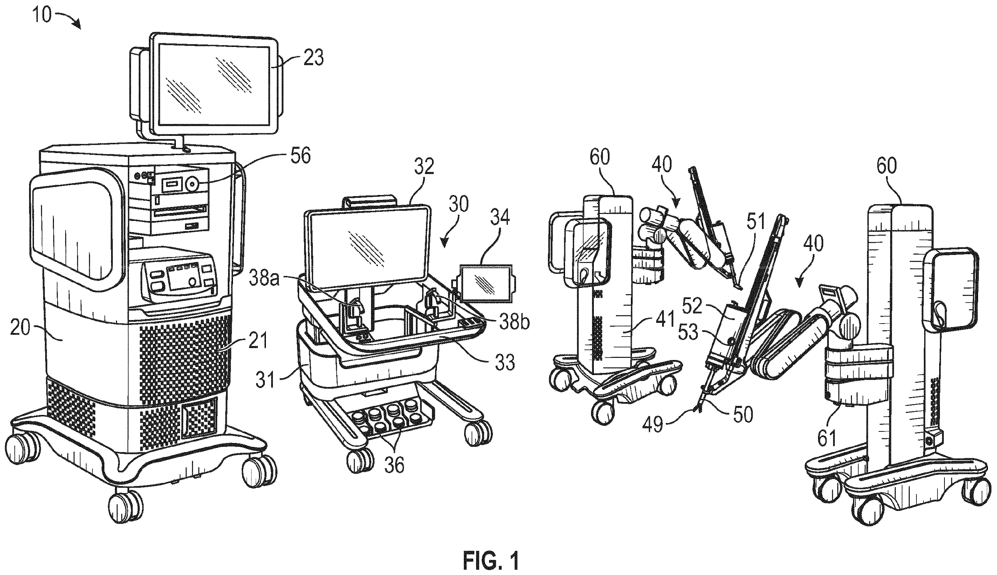

With reference to FIG. 1, a surgical robotic system 10 includes a control tower 20, which is connected to all of the components of the surgical robotic system 10 including a surgeon console 30 and one or more movable carts 60. Each of the movable carts 60 includes a robotic arm 40 having a surgical instrument 50 coupled thereto. The robotic arms 40 also couple to the movable carts 60. The robotic system 10 may include any number of movable carts 60 and/or robotic arms 40.

The surgical instrument 50 is configured for use during minimally invasive surgical procedures. In embodiments, the surgical instrument 50 may be configured for open surgical procedures. In further embodiments, the surgical instrument 50 may be an electrosurgical forceps configured to seal tissue by compressing tissue between jaw members and applying electrosurgical current thereto. In yet further embodiments, the surgical instrument 50 may be a surgical stapler including a pair of jaws configured to grasp and clamp tissue while deploying a plurality of tissue fasteners, e.g., staples, and cutting stapled tissue. In yet further embodiments, the surgical instrument 50 may be a surgical clip applier including a pair of jaws configured apply a surgical clip onto tissue.

One of the robotic arms 40 may include an endoscopic camera 51 configured to capture video of the surgical site. The endoscopic camera 51 may be a stereoscopic endoscope configured to capture two side-by-side (i.e., left and right) images of the surgical site to produce a video stream of the surgical scene. The endoscopic camera 51 is coupled to a video processing device 56, which may be disposed within the control tower 20. The video processing device 56 may be any computing device as described below configured to receive the video feed from the endoscopic camera 51 and output the processed video stream.

The surgeon console 30 includes a first display 32, which displays a video feed of the surgical site provided by camera 51 disposed on the robotic arm 40, and a second display 34, which displays a user interface for controlling the surgical robotic system 10. The first display 32 and second display 34 may be touchscreens allowing for displaying various graphical user inputs.

The surgeon console 30 also includes a plurality of user interface devices, such as foot pedals 36 and a pair of handle controllers 38a and 38b which are used by a user to remotely control robotic arms 40. The surgeon console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the handle controllers 38a and 38b. The foot pedals 36 may be used to enable and lock the hand controllers 38a and 38b, repositioning camera movement and electrosurgical activation/deactivation. In particular, the foot pedals 36 may be used to perform a clutching action on the hand controllers 38a and 38b. Clutching is initiated by pressing one of the foot pedals 36, which disconnects (i.e., prevents movement inputs) the hand controllers 38a and/or 38b from the robotic arm 40 and corresponding instrument 50 or camera 51 attached thereto. This allows the user to reposition the hand controllers 38a and 38b without moving the robotic arm(s) 40 and the instrument 50 and/or camera 51. This is useful when reaching control boundaries of the surgical space.

Each of the control tower 20, the surgeon console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-1203 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

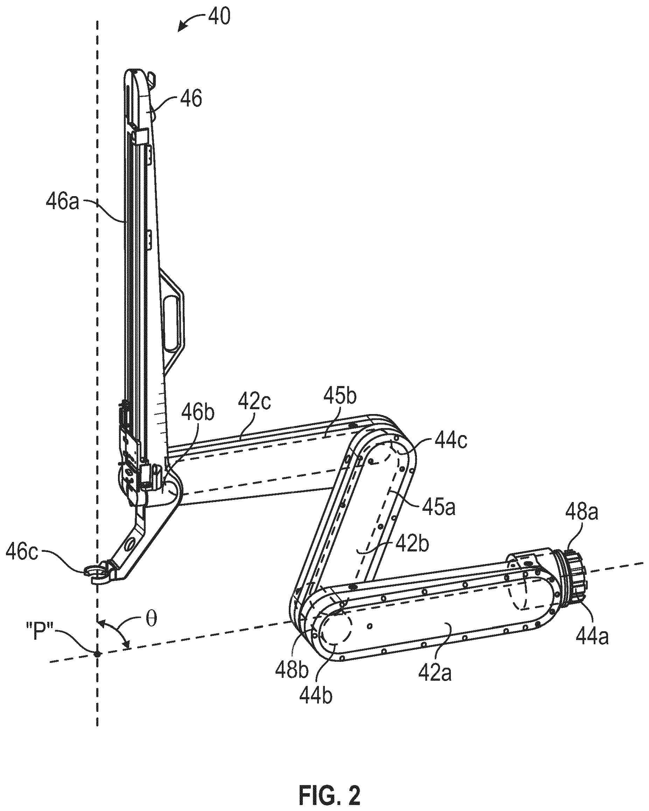

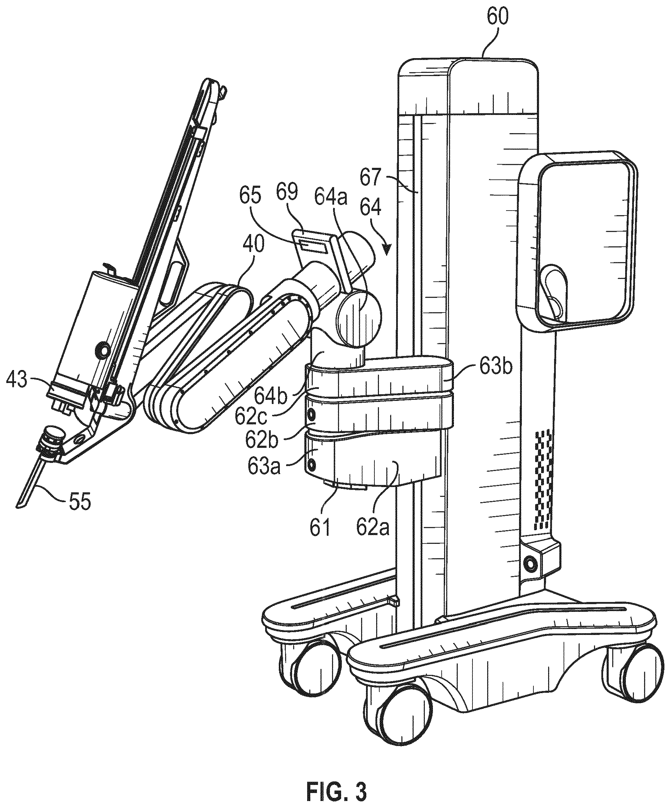

With reference to FIG. 2, each of the robotic arms 40 may include a plurality of links 42a, 42b, 42c, which are interconnected at joints 44a, 44b, 44c, respectively. Other configurations of links and joints may be utilized as known by those skilled in the art. The joint 44a is configured to secure the robotic arm 40 to the movable cart 60 and defines a first longitudinal axis. With reference to FIG. 3, the movable cart 60 includes a lift 67 and a setup arm 61, which provides a base for mounting of the robotic arm 40. The lift 67 allows for vertical movement of the setup arm 61. The movable cart 60 also includes a display 69 for displaying information pertaining to the robotic arm 40. In embodiments, the robotic arm 40 may include any type and/or number of joints.

The setup arm 61 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 61 includes controls 65 for adjusting movement of the links 62a, 62b, 62c as well as the lift 67. In embodiments, the setup arm 61 may include any type and/or number of joints.

The third link 62c may include a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46b via the belt 45b. Joint 44c may include a transfer case coupling the belts 45a and 45b, such that the actuator 48b is configured to rotate each of the links 42b, 42c and a holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. In other words, the pivot point "P" is a remote center of motion (RCM) for the robotic arm 40. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In embodiments, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the holder 46 defines a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components an end effector 49 of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During endoscopic procedures, the instrument 50 may be inserted through an endoscopic access port 55 (FIG. 3) held by the holder 46. The holder 46 also includes a port latch 46c for securing the access port 55 to the holder 46 (FIG. 2).

The IDU 52 is attached to the holder 46, followed by a sterile interface module (SIM) 43 being attached to a distal portion of the IDU 52. The SIM 43 is configured to secure a sterile drape (not shown) to the IDU 52. The instrument 50 is then attached to the SIM 43. The instrument 50 is then inserted through the access port 55 by moving the IDU 52 along the holder 46. The SIM 43 includes a plurality of drive shafts configured to transmit rotation of individual motors of the IDU 52 to the instrument 50 thereby actuating the instrument 50. In addition, the SIM 43 provides a sterile barrier between the instrument 50 and the other components of robotic arm 40, including the IDU 52.

The robotic arm 40 also includes a plurality of manual override buttons 53 (FIG. 1) disposed on the IDU 52 and the setup arm 61, which may be used in a manual mode. The user may press one or more of the buttons 53 to move the component associated with the button 53.

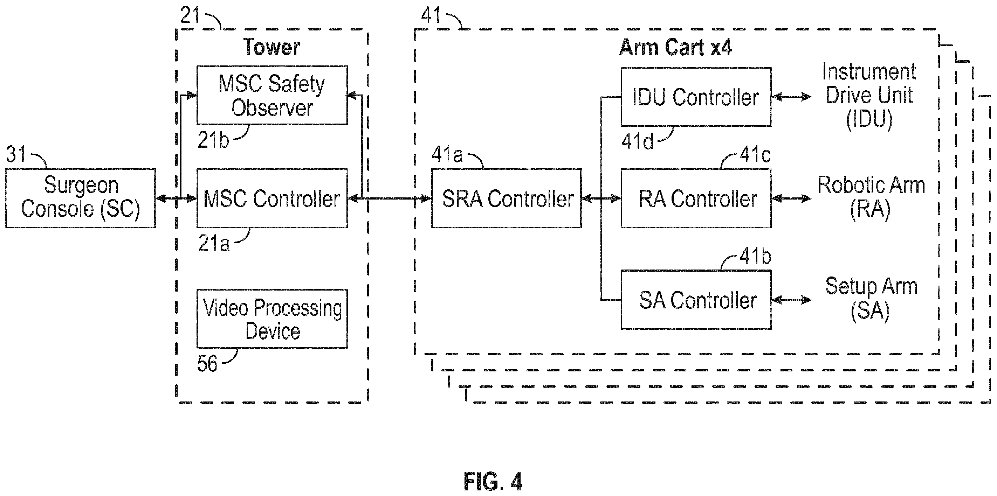

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgeon console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgeon console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

Each of joints 63a and 63b and the rotatable base 64 of the setup arm 61 are passive joints (i.e., no actuators are present therein) allowing for manual adjustment thereof by a user. The joints 63a and 63b and the rotatable base 64 include brakes that are disengaged by the user to configure the setup arm 61. The setup arm controller 41b monitors slippage of each of joints 63a and 63b and the rotatable base 64 of the setup arm 61, when brakes are engaged or can be freely moved by the operator when brakes are disengaged, but do not impact controls of other joints. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled in response to a pose of the handle controller controlling the robotic arm 40, e.g., the handle controller 38a, which is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controllers 38a may be embodied as a coordinate position and roll-pitch-yaw (RPY) orientation relative to a coordinate reference frame, which is fixed to the surgeon console 30. The desired pose of the instrument 50 is relative to a fixed frame on the robotic arm 40. The pose of the handle controller 38a is then scaled by a scaling function executed by the controller 21a. In embodiments, the coordinate position may be scaled down and the orientation may be scaled up by the scaling function. In addition, the controller 21a may also execute a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

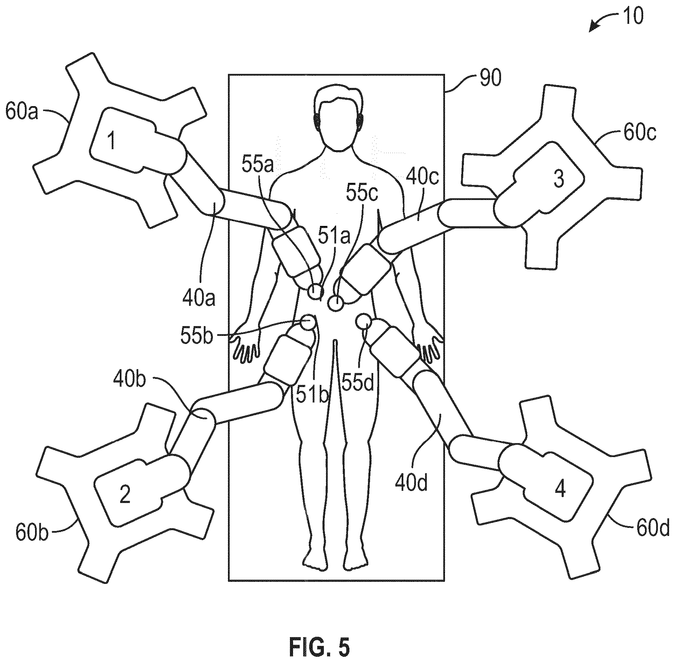

With reference to FIG. 5, the surgical robotic system 10 is setup around a surgical table 90. The system 10 includes movable carts 60a-d, which may be numbered "1" through "4." During setup, each of the carts 60a-d are positioned around the surgical table 90. Position and orientation of the carts 60a-d depends on a plurality of factors, such as placement of a plurality of access ports 55a-d, which in turn, depends on the surgery being performed. Once the port placements are determined, the access ports 55a-d are inserted into the patient, and carts 60a-d are positioned to insert instruments 50 and the endoscopic camera 51 into corresponding ports 55a-d.

During use, each of the robotic arms 40a-d is attached to one of the access ports 55a-d that is inserted into the patient by attaching the latch 46c (FIG. 2) to the access port 55 (FIG. 3). The IDU 52 is attached to the holder 46, followed by the SIM 43 being attached to a distal portion of the IDU 52. Thereafter, the instrument 50 is attached to the SIM 43. The instrument 50 is then inserted through the access port 55 by moving the IDU 52 along the holder 46.

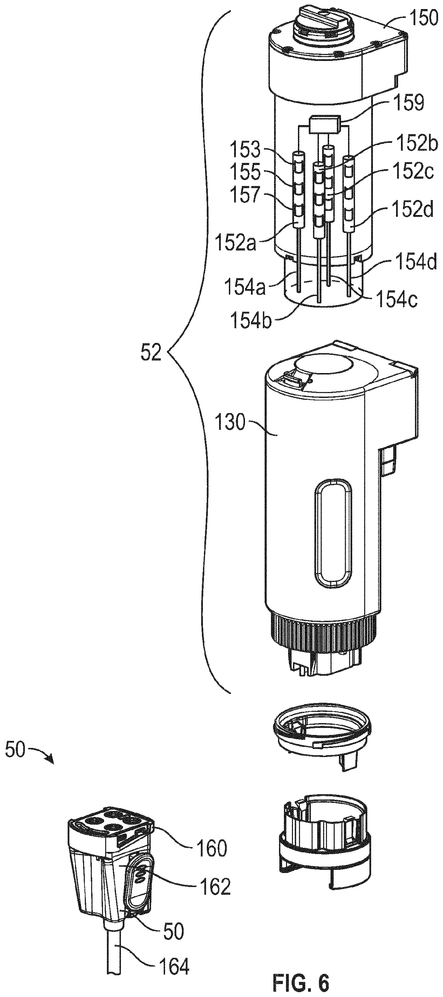

With reference to FIG. 6, the IDU 52 is shown in more detail and is configured to transfer power and actuation forces from its motors 152a, 152b, 152c, 152d to the instrument 50 to drive movement of components of the instrument 50, such as articulation, rotation, pitch, yaw, clamping, cutting, etc. The IDU 52 may also be configured for the activation or firing of an electrosurgical energy-based instrument or the like (e.g., cable drives, pulleys, friction wheels, rack and pinion arrangements, etc.).

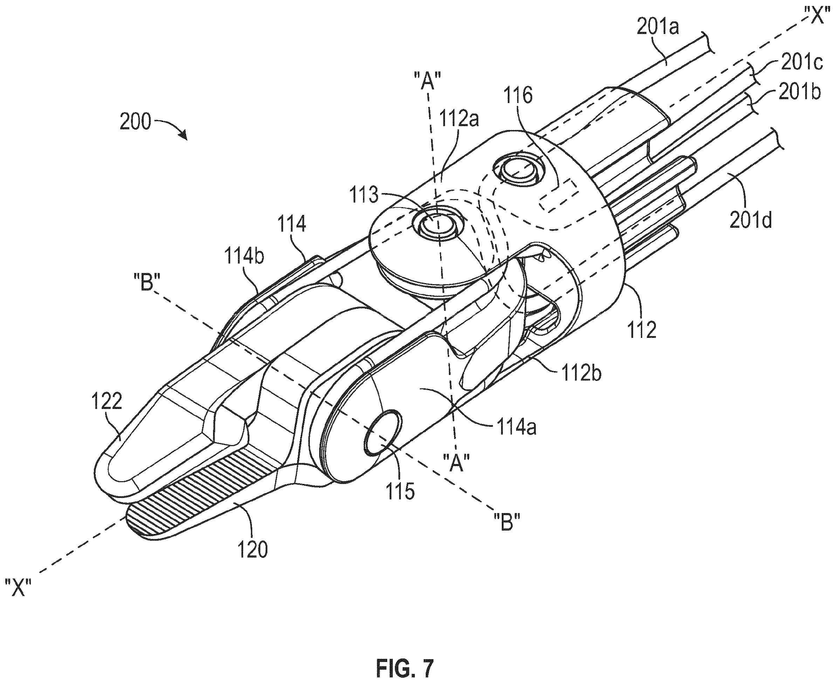

The IDU 52 includes a motor pack 150 and a sterile barrier housing 130. Motor pack 150 includes motors 152a, 152b, 152c, 152d for controlling various operations of the instrument 50. The instrument 50 is removably couplable to IDU 52. As the motors 152a, 152b, 152c, 152d of the motor pack 150 are actuated, rotation of the drive transfer shafts 154a, 154b, 154c, 154d of the motors 152a, 152b, 152c, 152d, respectively, is transferred to the drive assemblies of the instrument 50. The instrument 50 is configured to transfer rotational forces/movement supplied by the IDU 52 (e.g., via the motors 152a, 152b, 152c, 152d of the motor pack 150) into longitudinal movement or translation of the cables or drive shafts to effect various functions of an end effector 200 (FIG. 7).

Each of the motors 152a, 152b, 152c, 152d includes a current sensor 153, a torque sensor 155, and a position sensor 157. For conciseness only operation of the motor 152a is described below. The sensors 153, 155, 157 monitor the performance of the motor 152a. The current sensor 153 is configured to measure the current draw of the motor 152a and the torque sensor 155 is configured to measure motor torque. The torque sensor 155 may be any force or strain sensor including one or more strain gauges configured to convert mechanical forces and/or strain into a sensor signal indicative of the torque output by motor 152a. Position sensor 157 may be any device that provides a sensor signal indicative of the number of rotations of the motor 152a, such as a mechanical encoder or an optical encoder. Parameters which are measured and/or determined by position sensor 157 may include speed, distance, revolutions per minute, position, and the like. The sensor signals from sensors 153, 155, 157 are transmitted to the IDU controller 41d, which then controls the motors 152a, 152b, 152c, 152d based on the sensor signals. In particular, the motors 152a, 152b, 152c, 152d are controlled by an actuator controller 159, which controls torque outputted and angular velocity of the motors 152a, 152b, 152c, 152d. In embodiments, additional position sensors may also be used, which include, but are not limited to, potentiometers coupled to movable components and configured to detect travel distances, Hall Effect sensors, accelerometers, and gyroscopes. In embodiments, a single controller can perform the functionality of the IDU controller 41d and the actuator controller 159.

With reference to FIG. 6, instrument 50 includes an adapter 160 having a housing 162 at a proximal end portion thereof and an elongated shaft 164 that extends distally from housing 162. Housing 162 of instrument 50 is configured to selectively couple to IDU 52 of robotic, to enable motors 152a, 152b, 152c, 152d of IDU 52 to operate the end effector 200 of the instrument 50. Housing 162 of instrument 50 supports a drive assembly that mechanically and/or electrically cooperates with motors 152a, 152b, 152c, 152d of IDU 52. Drive assembly of instrument 50 may include any suitable electrical and/or mechanical component to effectuate driving force/movement.

The surgical instrument also includes an end effector 200 coupled to the elongated shaft 164. The end effector 200 may include any number of degrees of freedom allowing the end effector 200 to articulate, pivot, etc., relative to the elongated shaft 164. The end effector 200 may be any suitable surgical end effector configured to treat tissue, such as a dissector, grasper, sealer, stapler, etc.

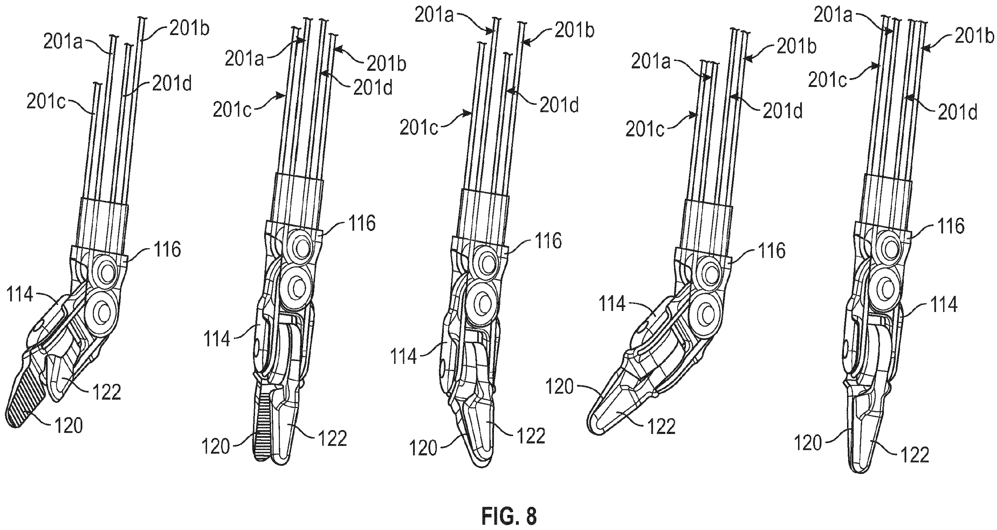

As shown in FIGS. 7 and 8, the end effector 200 may include a pair of opposing jaws 120 and 122 that are movable relative to each other. In embodiments, the end effector 200 may include a proximal portion 112 having a first pin 113 and a distal portion 114. Although the jaws 120 and 122 are shown as gripping jaws, it should be understood that the jaws may be any suitable type of jaw, such as shears, etc. The end effector 200 may be actuated using a plurality of cables 201a-d routed through proximal and distal portions 112 and 114 around their respective pulleys 112a, 112b, 114a, 114b, which are integrally formed as arms of the proximal and distal portions 112 and 114. Each of the cables 201a-d is actuated by a respective motor 152a-d via corresponding couplers disposed in adapter 160. In embodiments, the end effector 200, namely, the distal portion 114 and the jaws 120 and 122, may be articulated about the axis "A-A" to control a yaw angle of the end effector with respect to a longitudinal axis "X-X". The distal portion 114 includes a second pin 115 with a pair of jaws including a first jaw 120 and a second jaw 122 pivotably coupled to the second pin 115. The jaws 120 and 122 are configured to pivot about an axis "B-B" defined by the second pin 115 allowing for controlling a pitch angle of the jaws 120 and 122 as well as opening and closing the jaws 120 and 122. The yaw, pitch, and jaw angles between the jaws 120 and 122 as they are moved between open and closed positions are controlled by adjusting the tension and/or length and direction (e.g., proximal or distal) of the cables 201a-d as shown in FIG. 8. The end effector 200 also includes a cable displacement sensor 116 configured to measure position of the cables 201. Thus, the end effector 200 may have three degrees of freedom, yaw, pitch, and jaw angle between jaws 120 and 122.

Wristed end effector 200 utilizes for drive cables 201a-d to articulate pitch, yaw, and jaw degrees of freedom. The cables responsible for closing the jaws are called high-side cables 201b and 201c and those responsible for opening the jaws are called low-side cables 201a and 201d. Thus, the high-side cable 201c and low-side cable 201a actuate the second jaw 122, and high-side cable 201b and the low-side cable 201d actuate the first jaw 120. During closure, the high-side cables 201b and 201c are tensioned while minimum tension is applied to the low-side cables 201a and 201d. During opening, the tension is applied to the cables in reverse, i.e., higher tension to the low-side cables 201a and 201d and minimal tension to the high-side cables 201b and 201c. The cables 201a-d are controlled by their respective motors 152a-d. Thus, the motors 152b and 152c are high-side motors as they actuate high-side cables 201b and 201c and the motors 152a and 152d are low-side motors as they actuate low-side cables 201a and 201d.

The cables 201a-d are nominally under tension during tele-operation. Occasionally, the cables 201a-d may fail, e.g., due to wear. During certain movements of the end effector 200, such as closing of the jaws 120 and 122, when high tension is applied to the cables 201a-d some of the cables 201a-d may snap. With particular reference to the present design and routing of the cables 201a-d, high-side cables 201b, 201c may snap during high tension events. Following the snap, there is uncontrolled movement of the end effector 200, e.g., snapping to one side, due to high tension in the intact high side cable 201b or 201c which continues to actuate the corresponding motor 152b or 152c until the commanded position is reached. The present disclosure provides a system and a method configured to predict and/or detect when any of the cables 201a-d, and in particular high side cables 201b and 201c will snap using sensor signals from the motors 152a-d.

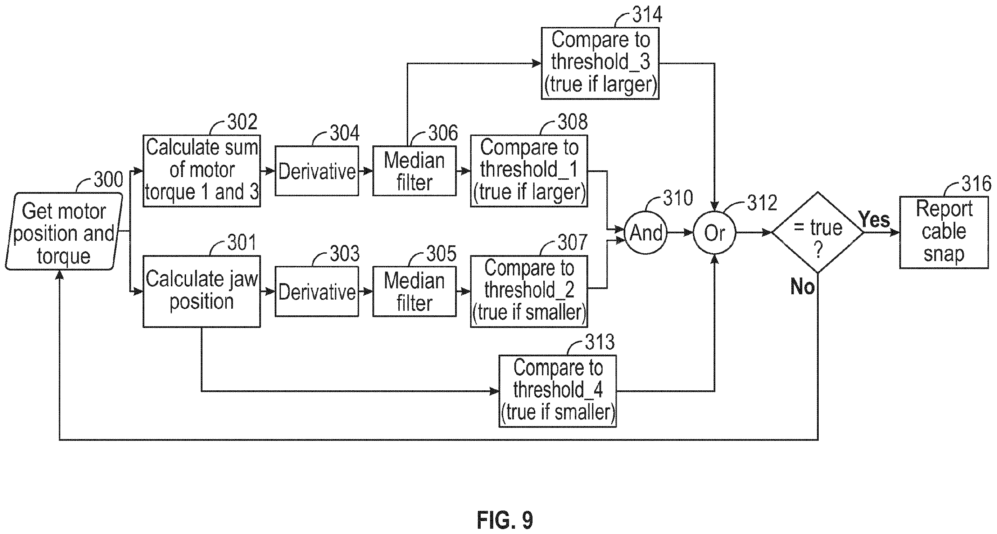

With reference to FIG. 9, a method for predicting or detecting breakage of high-side cables 201b and 201c is implemented as software instructions executable by any suitable processor of the robotic system 10, such as the IDU controller 41d, the main controller 21a, etc. At step 300, motor position and torque for each motor 152a-d are provided to the controller 41d. In particular, the torque sensor 155 provides torque measurements and the position sensor 157 provides angular rotational position of each of the motors 152a-d. The angular position is then used by the controller 41d to determine position of the cables 201a-d and the end effector 200. Steps 301-307, which are used for jaw position or angle verification, and steps 302-308, which are used for torque verification, may be executed in parallel. In particular, torque and jaw position are used to detect and/or predict cable breakage.

At step 301, the jaw position, e.g., angle between the jaws 120 and 122, is calculated based on the angular position data provided by the position sensors 157 of the motors 152a-d. The calculated jaw angle is then processed by taking a derivative thereof at step 303, which is then filtered using a median filter at step 305. The resulting filtered derivative of the jaw angle is then compared to a first torque threshold at step 307, which outputs a true value if the processed jaw angle value is smaller than the threshold, otherwise the controller 41d outputs a false value.

The method also performs torque verification at steps 302-308. At step 302 torque for motors 152b and 152c, which control the high-side cables 201b and 201c is summed to obtain a combined torque value. The resulting sum of the motor torques is then processed by taking a derivative thereof at step 304, which is then filtered using a median filter at step 306. The resulting filtered derivative of the summed motor torques is then compared to a first torque threshold at step 308, which outputs a true value if the processed torque sum value is larger than the threshold, otherwise the controller 41d outputs a false value.

At step 310, the controller receives as inputs the outputs from the torque and jaw position determinations of steps 307 and 308 and performs an AND operation on the inputs. Thus, if both values are true, i.e., the torque value is above the torque threshold and the jaw angle value is below the angle threshold, then the method proceeds to step 312. If the output of step 310 is no (i.e., one or both of the values of steps 307 and 308 is false), the method still proceeds to step 312.

Step 313 provides another layer of detection, which receives input from alternative, branching verification steps 313 and 314. At step 313, the controller 41d receives unprocessed jaw angle from step 301 and compares the jaw angle to a second jaw angle threshold. If the jaw angle is less than the second jaw angle threshold, then the controller 41d outputs a true value, otherwise the controller 41d outputs a false value.

At step 314, the controller 41d receives the processed motor torque value (i.e., filtered derivative of the summed torque) from step 306 and compares the torque value to a second torque threshold. If the torque value is larger than the second torque threshold, then the controller 41d outputs a true value, otherwise the controller 41d outputs a false value.

Thus, at step 312, the controller 41d compares three inputs from steps 310, 313, 314, and performs an OR operation on the inputs. Thus, if any of the inputs are true, cable breakage is detected. At step 316, the controller 41d reports the detected cable breakage to the controller 21a, which outputs the cable breakage of one of the high-side cables 201b and 201c on a GUI of one or more of the displays 23, 32, 34. In addition, the controller 21a may also take corrective action controlling the instrument 50, which may include adjusting the motors, e.g., lowering torque output or velocity, or powering off and stopping motors coupled to the broken cable, etc., overwriting user input where requested user inputs through the handle controllers 38a and 38b are not controlling the instrument, and/or automatically retracting the instrument 50 from the patient.

The present disclosure also provides for a method to detect a snap in low-side cables 201a and 201d. The IDU controller 41d executes a minimum torque algorithm which maintains tension in the cables 201a-d. Since there is no tension in one of the low-side cables 201a and 201d when one of them snaps, the minimum torque algorithm keeps increasing the commanded torque to remove slack in the broken cable. This results in unexpectedly high readings from the torque sensor 155 of the motors 152b and 152c actuating the high-side cables 201b and 201c.

As noted above, the low-side cables 201a and 201d are primarily used during opening of the jaws 120 and 122. Thus, when one of the low-side cables 201a and 201d snaps, the opening of the jaws 120 and 122 may not occur. Therefore, even though the IDU controller 41d may indicate fully open jaws 120 and 122, based on the positional feedback from the corresponding position sensors 157 of the motors 152a-d, the jaws 120 and 122 remain fully closed. Thus, when a new closing event is commanded, the IDU controller 41d charges the overstroke load on the fully closed blades. This results in an even higher torque sensor readings from the torque sensor 155 of the motors 152b and 152c actuating the high-side cables 201b and 201c. Thus, breakage of low-side cables 201a and 201d is detected during the subsequent closure event by using torque of the high-side motors 152b and 152c.

Thus, breakage of low-side cables 201a and 201d is reflected in the torque sensor readings from the torque sensor 155 of the high-side motors 152b and 152c actuating the high-side cables 201b and 201c. The sum of the torque sensor readings for motors 152b and 152c actuating the high-side cables 201b and 201c shows an increase in magnitude after an opening event of the jaws 120 and 122 and does not decrease to its expected range. Therefore, breakage of low-side cables 201a and 201d may be detected based on unexpectedly high torque readings in high-side cables 201b and 201c. The torque sensor readings for motors 152b and 152c actuating the high-side cables 201b and 201c are in general below a preset torque threshold, which may be about 0.35 Nm, except during closing events and an interval after each closing events until the unsaturated jaw position signal is settled.

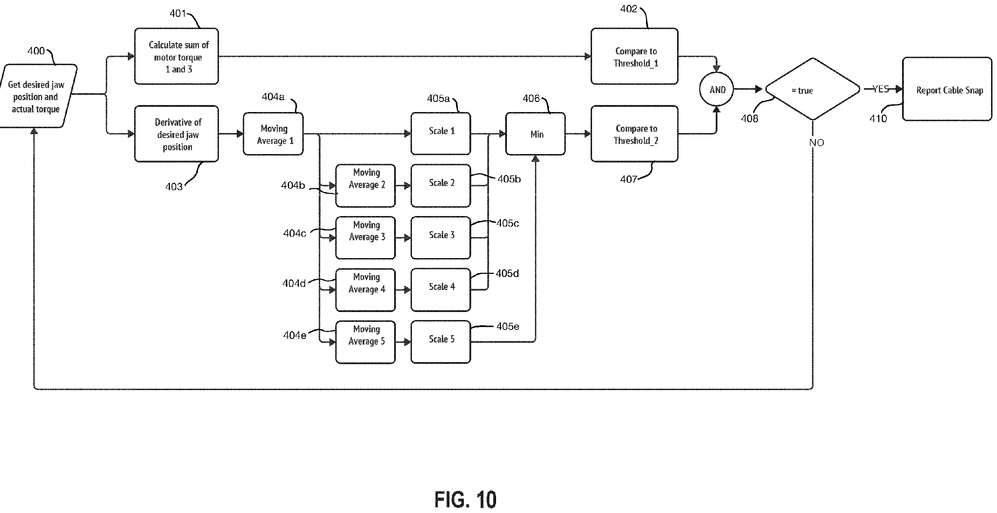

With reference to FIG. 10, a method for predicting or detecting breakage of low-side cables 201a and 201d is implemented as software instructions executable by any suitable processor of the robotic system 10, such as the IDU controller 41d, the main controller 21a, etc. At step 400, motor position and torque for each motor 152a-d are provided to the controller 41d. In particular, the torque sensor 155 provides torque measurements and the position sensor 157 provides angular rotational position of each of the motors 152a-d. The angular position is then used by the controller 41d to determine position of the cables 201a-d and by extension the end effector 200. Steps 401 and 402, which are used for torque verification, and steps 403-407, which are used for jaw position or angle verification, may be executed in parallel. In particular, torque and jaw position are used to detect and/or predict cable breakage.

The method performs torque verification at steps 401 and 402. At step 401 torque for motors 152b and 152c, which control the high-side cables 201b and 201c, is summed to obtain combined torque value. The resulting sum of the motor torques is compared to a first combined torque threshold at step 402, which outputs a true value if the processed torque sum value exceeds the threshold, otherwise the controller 41d outputs a false value.

At step 403, the jaw position, e.g., angle between the jaws 120 and 122, is calculated based on the angular position data provided by the position sensors 157 of the motors 152a-d. The calculated jaw angle is then processed by taking a derivative thereof. The derivative of the desired jaw position is utilized in the low-side cable snap detection algorithm to identify the closing events.

At step 404a a first moving average file is applied to the derivative of the jaw angle, i.e., position. At steps 404b-e multiple different moving average filters are applied to the output of the first moving average filter from step 404a. Multiple moving average filters of the derivative of the desired jaw position with different, i.e., unique, window sizes are performed at steps 404b-e and are used to handle the interval after each closing event in which the high-torque sensor readings are expected. The calculated moving averages from steps 404a-e are scaled at steps 405a-e, respectively. At step 406, a minimum function is performed on the scaled moving average values from steps 405a-e to identify the smallest value from the group. The smallest moving average of the derivative is then compared to a jaw position derivative threshold at step 407. The IDU controller 41d outputs a true value if the smallest moving average of the derivative exceeds the threshold, otherwise the controller 41d outputs a false value.

At step 408, the IDU controller 41d determines whether outputs of comparisons from steps 402 and 407 are true. If both are true, then at step 410 the IDU controller 41d reports the detected cable breakage to the controller 21a, which outputs the cable breakage of one of the low-side cables 201a and 201d on a GUI of one or more of the displays 23, 32, 34. In addition, the controller 21a may also take corrective action controlling the instrument 50, which may include adjusting the motors, e.g., lowering torque output or velocity, or powering off and stopping motors coupled to the broken cable, etc., overwriting user input where requested user inputs through the handle controllers 38a and 38b are not controlling the instrument, and/or automatically retracting the instrument 50 from the patient. If one or both are false, then the method returns to step 400 to continue monitoring breakage of low-side cables 201a and 201d.

It will be understood that various modifications may be made to the embodiments disclosed herein. Therefore, the above description should not be construed as limiting, but merely as exemplifications of various embodiments. Those skilled in the art will envision other modifications within the scope of the claims appended thereto.

## Claims

1. A surgical robotic system (10) comprising:
an instrument drive unit (52) including a plurality of motors (152a, 152b, 152c, 152d) including a first pair of motors (152b, 152c) and a second pair of motors (152a, 152d), wherein each motor of the plurality of motors includes a torque sensor (155) for measuring motor torque and a position sensor (157) for measuring angular position;
an instrument (50) coupled to the instrument drive unit, the instrument including:
an end effector (200) having a first jaw (120) and a second jaw (1220) each movable between an open position and a closed position;
a first pair of cables (201b, 201c) each of which is actuated by one motor of the first pair of motors to move the first jaw and the second jaw toward the closed position,
wherein one cable of the first pair of cables is coupled to the first jaw and another cable of the first pair of cables is coupled to the second jaw; and
a second pair of cables (201a, 201d) each of which is actuated by one motor of the second pair of motors to move the first jaw and the second jaw toward the open position,
wherein one cable of the second pair of cables is coupled to the first jaw and another cable of the second pair of cables is coupled to the second jaw; and
a controller (41d) adapted to:
calculate an angle between the first jaw and the second jaw based on the angular position of at least one motor of the plurality of motors;
calculate a combined torque of the first pair of motors;
determine whether at least one cable of the second pair of cables is broken based on the angle between the first jaw and the second jaw and the combined torque; and
output an alert based on the determination that at least one cable of the second pair of cables is broken.

2. The surgical robotic system according to claim 1, wherein the controller further calculates a derivative of the angle between the first jaw and the second jaw.

3. The surgical robotic system according to claim 2, wherein the controller further calculates a first moving average of the derivative of the angle between the first jaw and the second jaw and a plurality of subsequent moving averages of the first moving average.

4. The surgical robotic system according to claim 3, wherein the controller further calculates a scaled value for the first moving average and each moving average of the plurality of subsequent moving averages.

5. The surgical robotic system according to claim 3, wherein each moving average of the plurality of subsequent moving averages has a unique window size.

6. The surgical robotic system according to claim 3, wherein the controller identifies a minimum moving average of the derivative from the first moving average and each moving average of the plurality of subsequent moving averages.

7. The surgical robotic system according to claim 6, wherein the controller compares the minimum moving average of the derivative to a threshold derivative of the angle between the first jaw and the second jaw.

8. The surgical robotic system according to claim 7, wherein the controller determines whether at least one cable of the second pair of cables is broken, based on the combined torque of the first pair of motors exceeding a torque threshold and the minimum moving average of the derivative exceeding the threshold derivative of the angle between the first jaw and the second jaw.

9. A surgical robotic system as claimed in claims 1 to 8 wherein the first pair of motors (152b, 152c) and second pair of motors (152a, 152d) comprises: a first high-side motor (152b) having a first high-side torque sensor for measuring a torque of the first high-side motor and a first high-side position sensor for measuring a position of the first high-side motor; a first low-side motor (152a) having a first low-side torque sensor for measuring a torque of the first low-side motor and a first low-side position sensor for measuring a position of the first low-side motor; a second high-side motor (152c) having a second high-side torque sensor for measuring a torque of the second high-side motor and a second high-side position sensor for measuring a position of the second high-side motor; and a second low-side motor (152d) having a second low-side torque sensor for measuring a torque of the second low-side motor and a second low-side position sensor for measuring a position of the second low-side motor;
and wherein the first pair of cables and second pair of cables comprises: a first high-side cable (201b) coupled to the first jaw and actuatable by the first high-side motor to move the first jaw toward the closed position; a first low-side cable (201a) coupled to the first jaw and actuatable by the first low-side motor to move the first jaw toward the open position; a second high-side cable (201c) coupled to the second jaw and actuatable by the second high-side motor to move the second jaw toward the closed position; and a second low-side cable (201d) coupled to the second jaw and actuatable by the second low-side motor to move the second jaw toward the open position.

10. The surgical robotic system according to claim 9, wherein the controller determines whether at least one of the first low-side cable or the second low-side cable is broken, based on the angle between the first jaw and the second jaw exceeding an angle threshold and the minimum moving average of the derivative exceeding the threshold derivative of the angle between the first jaw and the second jaw.

## Patentansprüche

1. Chirurgisches Robotersystem (10), umfassend:
eine Instrumentenantriebseinheit (52), die eine Vielzahl von Motoren (152a, 152b, 152c, 152d) einschließt, die ein erstes Paar von Motoren (152b, 152c) und ein zweites Paar von Motoren (152a, 152d) einschließt, wobei jeder Motor der Vielzahl von Motoren einen Drehmomentsensor (155) zum Messen des Motordrehmoments und einen Positionssensor (157) zum Messen der Winkelposition einschließt;
ein Instrument (50), das an die Instrumentenantriebseinheit gekoppelt ist, wobei das Instrument einschließt:
einen Endeffektor (200), der eine erste Backe (120) und eine zweite Backe (1220) aufweist, die jeweils zwischen einer offenen Position und einer geschlossenen Position bewegbar sind;
ein erstes Paar von Kabeln (201b, 201c), von denen jedes durch einen Motor des ersten Paars von Motoren betätigt wird, um die erste Backe und die zweite Backe zu der geschlossenen Position hin zu bewegen, wobei ein Kabel des ersten Paars von Kabeln mit der ersten Backe gekoppelt ist und ein anderes Kabel des ersten Paars von Kabeln mit der zweiten Backe gekoppelt ist; und
ein zweites Paar von Kabeln (201a, 201d), von denen jedes durch einen Motor des zweiten Paars von Motoren betätigt wird, um die erste Backe und die zweite Backe zu der offenen Position hin zu bewegen, wobei ein Kabel des zweiten Paars von Kabeln mit der ersten Backe gekoppelt ist und ein anderes Kabel des zweiten Paars von Kabeln mit der zweiten Backe gekoppelt ist; und
eine Steuerung (41d), die angepasst ist zum:
Berechnen eines Winkels zwischen der ersten Backe und der zweiten Backe basierend auf der Winkelposition mindestens eines Motors der Vielzahl von Motoren;
Berechnen eines kombinierten Drehmoments des ersten Paars von Motoren;
Bestimmen, ob mindestens ein Kabel des zweiten Paars von Kabeln gebrochen ist, basierend auf dem Winkel zwischen der ersten Backe und der zweiten Backe und dem kombinierten Drehmoment; und
Ausgeben einer Warnung basierend auf der Bestimmung, dass mindestens ein Kabel des zweiten Paars von Kabeln gebrochen ist.

2. Chirurgisches Robotersystem nach Anspruch 1, wobei die Steuerung ferner ein Derivat des Winkels zwischen der ersten Backe und der zweiten Backe berechnet.

3. Chirurgisches Robotersystem nach Anspruch 2, wobei die Steuerung ferner einen ersten gleitenden Mittelwert des Derivats des Winkels zwischen der ersten Backe und der zweiten Backe und eine Vielzahl von nachfolgenden gleitenden Mittelwerten des ersten gleitenden Mittelwerts berechnet.

4. Chirurgisches Robotersystem nach Anspruch 3, wobei die Steuerung ferner einen skalierten Wert für den ersten gleitenden Mittelwert und jeden gleitenden Mittelwert der Vielzahl von nachfolgenden gleitenden Mittelwerten berechnet.

5. Chirurgisches Robotersystem nach Anspruch 3, wobei jeder gleitende Mittelwert der Vielzahl von nachfolgenden gleitenden Mittelwerten eine eindeutige Fenstergröße aufweist.

6. Chirurgisches Robotersystem nach Anspruch 3, wobei die Steuerung einen minimalen gleitenden Mittelwert des Derivats aus dem ersten gleitenden Mittelwert und jedem gleitenden Mittelwert der Vielzahl von nachfolgenden gleitenden Mittelwerten identifiziert.

7. Chirurgisches Robotersystem nach Anspruch 6, wobei die Steuerung den minimalen gleitenden Mittelwert des Derivats mit einem Schwellenderivat des Winkels zwischen der ersten Backe und der zweiten Backe vergleicht.

8. Chirurgisches Robotersystem nach Anspruch 7, wobei die Steuerung bestimmt, ob mindestens ein Kabel des zweiten Paars von Kabeln gebrochen ist, basierend darauf, dass das kombinierte Drehmoment des ersten Paars von Motoren eine Drehmomentschwelle überschreitet und der minimale gleitende Mittelwert des Derivats das Schwellenderivat des Winkels zwischen der ersten Backe und der zweiten Backe überschreitet.

9. Chirurgisches Robotersystem nach den Ansprüchen 1 bis 8, wobei das erste Paar von Motoren (152b, 152c) und das zweite Paar von Motoren (152a, 152d) umfasst: einen ersten hochseitigen Motor (152b), der einen ersten hochseitigen Drehmomentsensor zum Messen eines Drehmoments des ersten hochseitigen Motors und einen ersten hochseitigen Positionssensor zum Messen einer Position des ersten hochseitigen Motors aufweist; einen ersten niedrigseitigen Motor (152a), der einen ersten niedrigseitigen Drehmomentsensor zum Messen eines Drehmoments des ersten niedrigseitigen Motors und einen ersten niedrigseitigen Positionssensor zum Messen einer Position des ersten niedrigseitigen Motors aufweist; einen zweiten hochseitigen Motor (152c), der einen zweiten hochseitigen Drehmomentsensor zum Messen eines Drehmoments des zweiten hochseitigen Motors und einen zweiten hochseitigen Positionssensor zum Messen einer Position des zweiten hochseitigen Motors aufweist; und einen zweiten niedrigseitigen Motor (152d), der einen zweiten niedrigseitigen Drehmomentsensor zum Messen eines Drehmoments des zweiten niedrigseitigen Motors und einen zweiten niedrigseitigen Positionssensor zum Messen einer Position des zweiten niedrigseitigen Motors aufweist;
und wobei das erste Paar von Kabeln und das zweite Paar von Kabeln umfasst: ein erstes hochseitiges Kabel (201b), das an die erste Backe gekoppelt ist und durch den ersten hochseitigen Motor betätigbar ist, um die erste Backe zu der geschlossenen Position hin zu bewegen; ein erstes niedrigseitiges Kabel (201a), das an die erste Backe gekoppelt ist und durch den ersten niedrigseitigen Motor betätigbar ist, um die erste Backe zu der offenen Position hin zu bewegen; ein zweites hochseitiges Kabel (201c), das an die zweite Backe gekoppelt ist und durch den zweiten hochseitigen Motor betätigbar ist, um die zweite Backe zu der geschlossenen Position hin zu bewegen; und ein zweites niedrigseitiges Kabel (201d), das an die zweite Backe gekoppelt ist und durch den zweiten niedrigseitigen Motor betätigbar ist, um die zweite Backe zu der offenen Position hin zu bewegen.

10. Chirurgisches Robotersystem nach Anspruch 9, wobei die Steuerung bestimmt, ob mindestens eines des ersten niedrigseitigen Kabels oder des zweiten niedrigseitigen Kabels gebrochen ist, basierend darauf, dass der Winkel zwischen der ersten Backe und der zweiten Backe eine Winkelschwelle überschreitet und der minimale gleitende Mittelwert des Derivats das Schwellenderivat des Winkels zwischen der ersten Backe und der zweiten Backe überschreitet.

## Revendications

1. Système robotique chirurgical (10) comprenant :
une unité d'entraînement d'instrument (52) comportant une pluralité de moteurs (152a, 152b, 152c, 152d) comportant une première paire de moteurs (152b, 152c) et une seconde paire de moteurs (152a, 152d), dans lequel chaque moteur de la pluralité de moteurs comporte un capteur de couple (155) permettant de mesurer le couple de moteur et un capteur de position (157) permettant de mesurer une position angulaire ;
un instrument (50) accouplé à l'unité d'entraînement d'instrument, l'instrument comportant :
un effecteur terminal (200) ayant une première mâchoire (120) et une seconde mâchoire (1220), chacune étant mobile entre une position ouverte et une position fermée ;
une première paire de câbles (201b, 201c) dont chacune est actionnée par un moteur de la première paire de moteurs pour déplacer la première mâchoire et la seconde mâchoire vers la position fermée, dans lequel un câble de la première paire de câbles est accouplé à la première mâchoire et un autre câble de la première paire de câbles est accouplé à la seconde mâchoire ; et
une seconde paire de câbles (201a, 201d) dont chacune est actionnée par un moteur de la seconde paire de moteurs pour déplacer la première mâchoire et la seconde mâchoire vers la position ouverte, dans lequel un câble de la seconde paire de câbles est accouplé à la première mâchoire et un autre câble de la seconde paire de câbles est accouplé à la seconde mâchoire ; et
un dispositif de commande (41d) destiné à :
calculer un angle entre la première mâchoire et la seconde mâchoire en fonction de la position angulaire d'au moins un moteur de la pluralité de moteurs ;
calculer un couple combiné de la première paire de moteurs ;
déterminer si au moins un câble de la seconde paire de câbles est rompu en fonction de l'angle entre la première mâchoire et la seconde mâchoire et du couple combiné ; et
émettre une alerte en fonction de la détermination qu'au moins un câble de la seconde paire de câbles est rompu.

2. Système robotique chirurgical selon la revendication 1, dans lequel le dispositif de commande calcule en outre une dérivée de l'angle entre la première mâchoire et la seconde mâchoire.

3. Système robotique chirurgical selon la revendication 2, dans lequel le dispositif de commande calcule en outre une première moyenne mobile de la dérivée de l'angle entre la première mâchoire et la seconde mâchoire et une pluralité de moyennes mobiles ultérieures de la première moyenne mobile.

4. Système robotique chirurgical selon la revendication 3, dans lequel le dispositif de commande calcule en outre une valeur mise à l'échelle pour la première moyenne mobile et chaque moyenne mobile de la pluralité de moyennes mobiles ultérieures.

5. Système robotique chirurgical selon la revendication 3, dans lequel chaque moyenne mobile de la pluralité de moyennes mobiles ultérieures a une taille de fenêtre unique.

6. Système robotique chirurgical selon la revendication 3, dans lequel le dispositif de commande identifie une moyenne mobile minimale de la dérivée à partir de la première moyenne mobile et chaque moyenne mobile de la pluralité de moyennes mobiles ultérieures.

7. Système robotique chirurgical selon la revendication 6, dans lequel le dispositif de commande compare la moyenne mobile minimale de la dérivée à une dérivée seuil de l'angle entre la première mâchoire et la seconde mâchoire.

8. Système robotique chirurgical selon la revendication 7, dans lequel le dispositif de commande détermine si au moins un câble de la seconde paire de câbles est rompu, en fonction du couple combiné de la première paire de moteurs dépassant un seuil de couple et de la moyenne mobile minimale de la dérivée dépassant la dérivée seuil de l'angle entre la première mâchoire et la seconde mâchoire.

9. Système robotique chirurgical selon les revendications 1 à 8, dans lequel la première paire de moteurs (152b, 152c) et la seconde paire de moteurs (152a, 152d) comprennent : un premier moteur côté haut (152b) ayant un premier capteur de couple côté haut pour mesurer un couple du premier moteur côté haut et un premier capteur de position côté haut pour mesurer une position du premier moteur côté haut ; un premier moteur côté bas (152a) ayant un premier capteur de couple côté bas pour mesurer un couple du premier moteur côté bas et un premier capteur de position côté bas pour mesurer une position du premier moteur côté bas ; un second moteur côté haut (152c) ayant un second capteur de couple côté haut pour mesurer un couple du second moteur côté haut et un second capteur de position côté haut pour mesurer une position du second moteur côte haut ; et un second moteur côté bas (152d) ayant un second capteur de couple côté bas pour mesurer un couple du second moteur côté bas et un second capteur de position côté bas pour mesurer une position du second moteur côté bas ;
et dans lequel la première paire de câbles et la seconde paire de câbles comprennent : un premier câble côté haut (201b) accouplé à la première mâchoire et pouvant être actionné par le premier moteur côté haut pour déplacer la première mâchoire vers la position fermée ; un premier câble côté bas (201a) accouplé à la première mâchoire et pouvant être actionné par le premier moteur côté bas pour déplacer la première mâchoire vers la position ouverte ; un second câble côté haut (201c) accouplé à la seconde mâchoire et pouvant être actionné par le second moteur côté haut pour déplacer la seconde mâchoire vers la position fermée ; et un second câble côté bas (201d) accouplé à la seconde mâchoire et pouvant être actionné par le second moteur côté bas pour déplacer la seconde mâchoire vers la position ouverte.

10. Système robotique chirurgical selon la revendication 9, dans lequel le dispositif de commande détermine si au moins l'un parmi le premier câble côté bas ou le second câble côté bas est rompu, en fonction de l'angle entre la première mâchoire et la seconde mâchoire dépassant un seuil d'angle et de la moyenne mobile minimale de la dérivée dépassant la dérivée seuil de l'angle entre la première mâchoire et la seconde mâchoire.
